# EUROPEAN PATENT APPLICATION

(11) **EP 4 501 346 A1**
(43) Date of publication of application: **05.02.2025**
(21) Application number: 23189833.9
(22) Date of filing: 04.08.2023
(51) Int. Cl.: A61K 38/18, A61K 8/04, A61P 25/00, A61P 27/16

(54) **METHOD FOR THE PREVENTION OF HEARING LOSS INDUCED BY A PLATINUM-BASED CHEMOTHERAPY DRUG**

(71) Applicant: Dompe' Farmaceutici S.P.A., 20122 Milan (IT)
(72) Inventor: BRANDOLINI, Laura, 67100 L'Aquila (IT); GIORGIO, Cristina, 67100 L'Aquila (IT); ARAMINI, Andrea, 67100 L'Aquila (IT); ALLEGRETTI, Marcello, 67100 L'Aquila (IT); DETTA, Nicola, 80131 Napoli (IT); ROMEO, Tiziana, 60100 L'Aquila (IT); FETONI, Anna Rita, 20123 Milano (IT)
(74) Representative: Dompé farmaceutici Spa

(57) **Abstract**

The present invention relates to brain derived nerve factor (BDNF) for use in the prevention of a hearing loss induced by a platinum-based chemotherapy drug in a subject, wherein said BDNF is administered intratympanically to said subject.

## Description

### FIELD OF THE INVENTION

The present invention relates to BDNF for use in preventing the onset of hearing loss in a subject receiving a platinum-based chemotherapy drug.

### BACKGROUND OF THE INVENTION

Cisplatin and other platinum-based chemotherapy drugs, including carboplatin and oxaliplatin, are widely used and effective drugs for the treatment of solid tumors, such as ovarian, cervical, testicular, non-small-cell lung, bladder, and head and neck cancers. Notwithstanding their therapeutic potential, the use of these agents may be associated to a number of adverse events, due to their toxic effects in different organs. In particular, accumulation in the ear is associated with ototoxic hearing loss, with is bilateral and permanent and severely affects the quality of life for cancer patients.

It has been reported that the incidence rate of cisplatin-induced ototoxicity in the population is 36% of adult patients and 40%-60% of pediatric patients (Chattaraj et al, JCO Oncol Pract 2023, 19: 278-283). In children, the consequences of hearing loss are particularly critical, as they are associated with delayed speech and language development, affecting literacy, academic achievement, and social and emotional development. In adults, it results in impaired social communication, lower income, poorer mental health and cognitive impairment.

The receptor organ for hearing is the organ of Corti, located in the cochlea. It is a specialized sensory epithelium, constituted by different cell types. The mechanosensory cells, known as hair cells (HCs), allow the conversion of sound-induced vibrations into electrochemical signals. They are organized in three rows of outer HCs (OHCs) and one row of inner HCs (IHCs), surrounded by non-sensory supporting cells (SCs) that play a major role in the maintenance of structural and functional properties of the sensory epithelium. Both HC types convert mechanical activation into an electrical signal, which is communicated via neurotransmitters (glutamate) to the primary auditory neurons, the Spiral Ganglion Neurons (SGNs), which convey the information to the central cochlear nucleus in the brainstem.

In platinum-based chemotherapy-induced hearing loss, HCs are the primary cells to degenerate, and their death is followed by a progressive degeneration of afferent SGNs, caused by the loss of neurotrophic support by HCs. In particular, it has been shown that cisplatin inflicts injuries mainly to outer hair cells, progressing from the third to the first row and to some extent to the inner hair cells of the organ of Corti in the basal turn of the cochlea, followed by alterations also in sensorial cells situated in the apex. Also supporting cells, marginal cells of the stria vascularis are targeted (Chirtes et al, BioMed Research International 2014, Article ID 925485).

A two-phase mechanism has been proposed for the induction of toxicity in hair cells by cisplatin and other platinum-based chemotherapy drugs. In a first, extracellular phase, cisplatin enters the inner ear through the blood-labyrinth barrier and accumulates herein, leading to creation of an abnormal endocochlear potential and disruption of cochlear endolymph homeostasis, that activate inflammatory responses of the outer hair cells. In a second phase, cisplatin enters the hair cells and causes damage to mitochondrial and nuclear DNA, redox system imbalance, and mitochondria and endoplasmic reticulum dysfunction, ultimately leading to various forms of programmed cell death (Wang X. at al, Biomedicine & Pharmacotherapy 2023, 157: 114045).

There is an urgent need the prevent the onset of drug-induced hearing loss in cancer patients receiving chemotherapy with platinum-based agents, without interfering with the chemotherapeutic treatment.

Given the complexity of the mechanisms that lead to cisplatin-induced hearing loss, this would require agents that are able to target the different mechanisms by which platinum-based drugs induce damage into the ear, in particular to the organ of Corti and to SGN. To this aim, it has been proposed in the literature the use of a combinatorial otoprotective approach with the administration of a cocktail of agents that are collectively able to inhibit the different pathways underlying cisplatin toxicity (Febles et al, Hear. Res. 2022, 415:108430; Zhang et al, Front. Cell. Neurosci. 2023, 17:1197051).

BDNF is a neurotrophic factor which is present in the cochlea and essential for inner ear development and survival. A number of studies have demonstrated, in vitro and ex vivo, that recombinant human BDNF is effective in protecting SGN from cell death following hair cell degeneration, induced by cisplatin or other ototoxic agents. However, BDNF has shown no effect on hair cell loss in the organ of Corti (Gabaizadeh et al. Molecular Brain Research 1997: 5071-78 1997, Tisi et al, Brain Sci. 2022, 12: 2; US 6,225,282).

In US 6,225,282 it is suggested the use of BDNF in the treatment of ototoxicity, in combination with interventions directed to re-establish hair cell functions.

### SUMMARY OF THE INVENTION

The present inventors have now surprisingly found that BDNF, when administered intratympanically is unexpectedly effective in the prevention of hearing loss induced by a platinum-based chemotherapy drug in a subject, resulting in the complete prevention of the onset of hearing loss induced by these drugs.

Accordingly, a first object of the invention is Brain-Derived Neurotrophic Factor (BDNF) for use in the prevention of a hearing loss induced by a platinum-based chemotherapy drug in a subject, wherein said BDNF is administered intratympanically to said subject.

A further object of the invention is a pharmaceutical composition for intratympanic administration comprising BDNF for use in the prevention of a hearing loss induced by a platinum-based chemotherapy drug in a subject, wherein said composition is administered intratympanically to said subject.

### BRIEF DESCRIPTION OF THE FIGURES

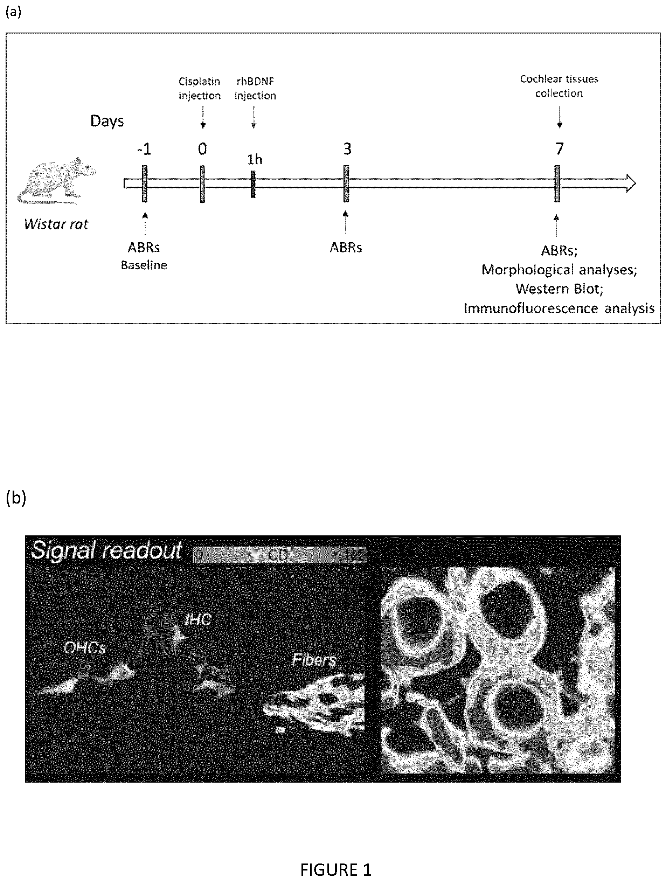

**Figure 1****:** Panel (a) shows a schematic outline of the experimental procedure on BDNF treated groups, as described in Example 2: Baseline hearing thresholds were evaluated by using auditory brainstem responses (ABR) the day before (-1) cisplatin treatment (0). One hour (1h) after cisplatin injection, rhBDNF was administered by intratympanic route. Functional analyses were performed by ABR assessment after 3 and 7 days from cisplatin administration, as described in Example 4 Cochlear tissues were collected after 7 days to perform experimental analyses, as described in Examples 3, 5, 6, 7 and 8. Panel (b) shows TrkB expression in cochlear structures (left image, where outer hair cells: OHCs; inner hair cells IHCs and afferent fibers Fibers) and Spiral Ganglion Neurons (SGN) (right image), as evidenced by immunofluorescence assay on cochlear sections, as described in Example 3.

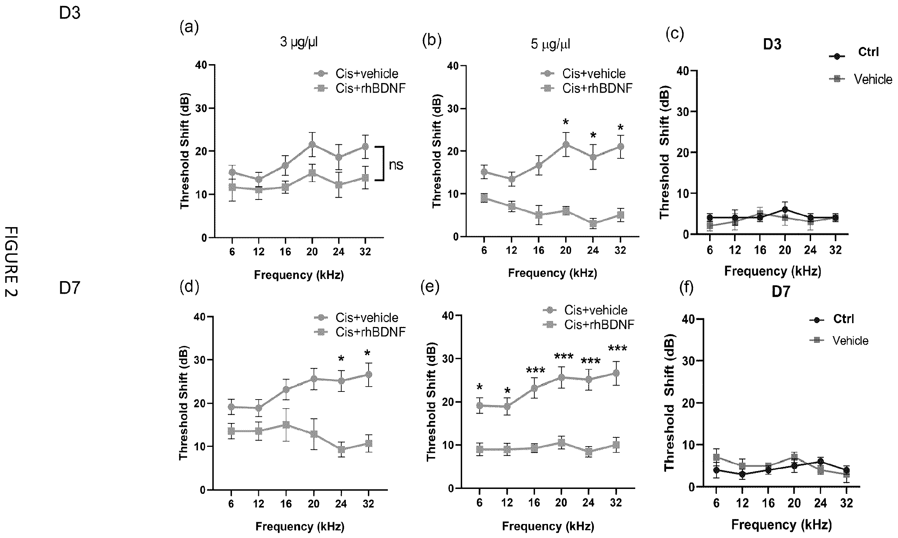

**Figure 2** show the results of the ABRs analysis carried out in Example 4. Panels (a) and (b) show the mean auditory threshold shift in Cis+vehicle and Cis+rhBDNF, at 3 µg/µl (panel a) and 5 µg/µl (panel b) groups, as described in Example 2, measured at 3 days after cisplatin administration (D3). (Two-Way Anova; 20 kHz, p= 0,0473; 24 kHz, p= 0,0457; 32 kHz, p= 0,0371). Panels (d) and (e) show the mean auditory threshold shift in Cis+vehicle and Cis+rhBDNF, at 3 µg/µl (panel d) and 5 µg/µl (panel e) groups, as described in Example 2, measured at 7 days after cisplatin administration (D3) D7. (Two-Way Anova; 24 kHz; p= 0,0344; 32 kHz, p= 0,0337). Asterisks indicate significant differences between groups (* p < 0.05; ** p < 0.01; *** p < 0.001. Panels (c) and (f) show mean auditory threshold shift measured in Ctrl and Vehicle groups, as described in Example 2, at 3 (panel (c)) and 7 (panel (f)) days after intratympanic injection.

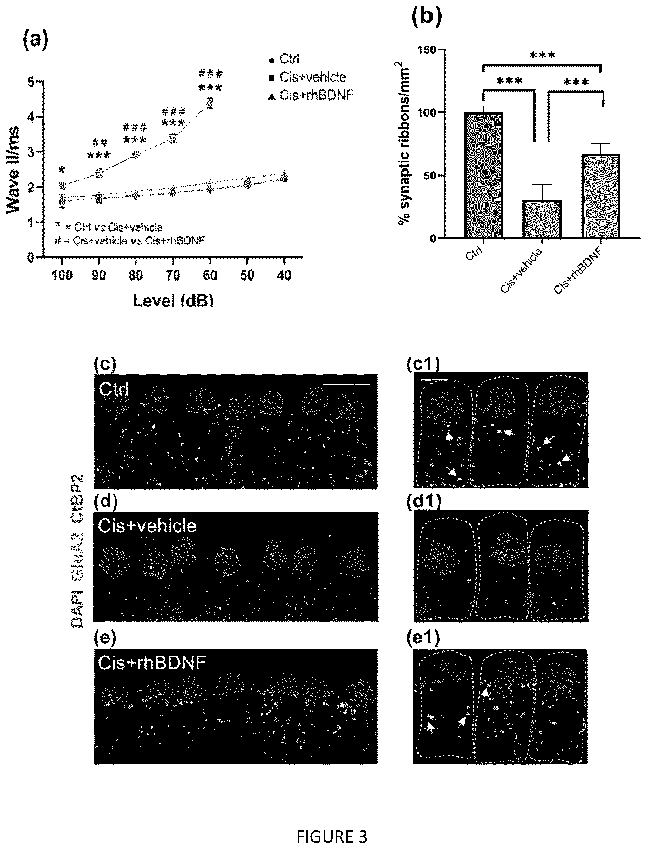

**Figure 3****:** panel (a) shows the latency-intensity (L-I) curve (mean ± SEM) of the ABR wave II (P2) at 24 kHz in Ctrl, Cis+vehicle and Cis+rhBNDF groups, as described in Example 2, measured as described in Example 4 (n=9 rats for each group; Two-Way Anova. 100 dB: Ctrl vs Cis+vehicle, p= 0,0175; 90 dB: Ctrl vs Cis+vehicle p< 0,0001; Cis+vehicle vs Cis+rhBDNF, p= 0,0022; 80 dB, 70 dB and 60 dB: Ctrl vs Cis+vehicle, p<0,0001; Cis+vehicle vs Cis+rhBDNF, p<0,0001). Panel (b) shows histograms (mean ± SEM) representing the count of paired synaptic ribbons expressed in percentage, measured according to the procedure of Example 5 in Ctrl, Cis+vehicle and Cis+rhBDNF groups as described in Example 2. Panels (c)-(e) show representative images of micro-dissected whole mount sections stained with pre-synaptic marker, CtBP2 (red channel), and post-synaptic marker, GluA2 (green channel) and double stained with DAPI in the IHC area of cochlear middle turn. In the Ctrl group (c, c1), pre-synaptic ribbons and post-synaptic receptors are clearly detected (white arrows in c1 indicate paired synaptic ribbons). In Cis+vehicle group (d, d1), a markedly decrease in synaptic contacts is showed. After rhBDNF administration (e, e1), an increase of synaptic contacts is observed (n=6 rats for each group; one-way Anova. Ctrl vs Cis+vehicle, p< 0,0001; Ctrl vs Cis+rhBDNF, p= 0,0003; Cis+vehicle vs Cis+rhBDNF p<0,0001).
   Scale bar in c: 10 µm, scale bar in c1: 2.5 µm. Asterisks indicate significant differences between groups (* p < 0.05; ** p < 0.01; *** p < 0.001).

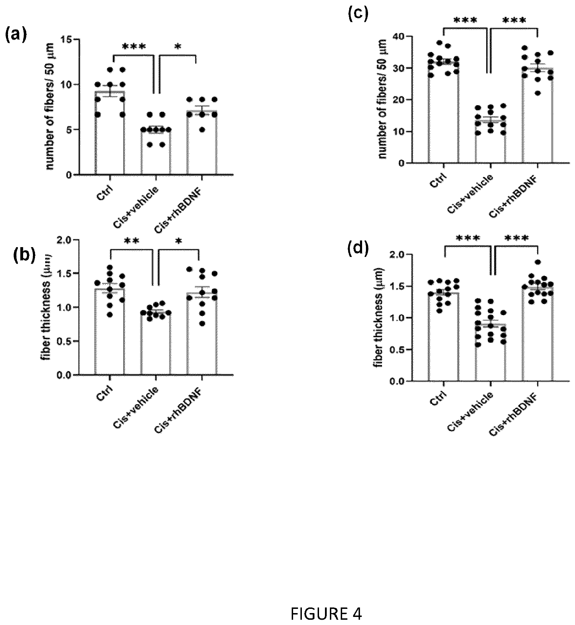

**Figure 4****:** Panels (a) and (c) show histograms representing the number of neural fibers (means ± SEM) between OHCs and SGNs (d, n=5 rats for each group; one-way Anova, Ctrl vs Cis+vehicle, p < 0,0001; Ctrl vs Cis+rhBDNF, p = 0,03; Cis+vehicle vs Cis+rhBDNF p = 0,02) (panel (a)) and between IHCs and SGNs (i, n=5 rats for each group; one-way Anova, Ctrl vs Cis+vehicle, p< 0,0001; Cis+vehicle vs Cis+rhBDNF p< 0,0001) (panel (c)) analyzed in a segment of 50 µm. Panels (b) and (d) show histograms representing the thickness (means ± SEM) of fibers between OHCs and SGNs (e, n=5 rats for each group; one-way Anova, Ctrl vs Cis+vehicle, p= 0,0026; Cis+vehicle vs Cis+rhBDNF p=0,012) (panel (b)) and between IHCs and SGNs (j, n=5 rats for each group; one-way Anova, Ctrl vs Cis+vehicle, p< 0,0001; Cis+vehicle vs Cis+rhBDNF p< 0,0001) (Panel (d)). OHCs: Outer Hair Cells; IHC: Inner Hair Cell; AFs: Afferent Fibers; SGNs: Spiral Ganglion Neurons. Asterisks indicate significant differences between groups (* p < 0.05; ** p < 0.01; *** p < 0.001).

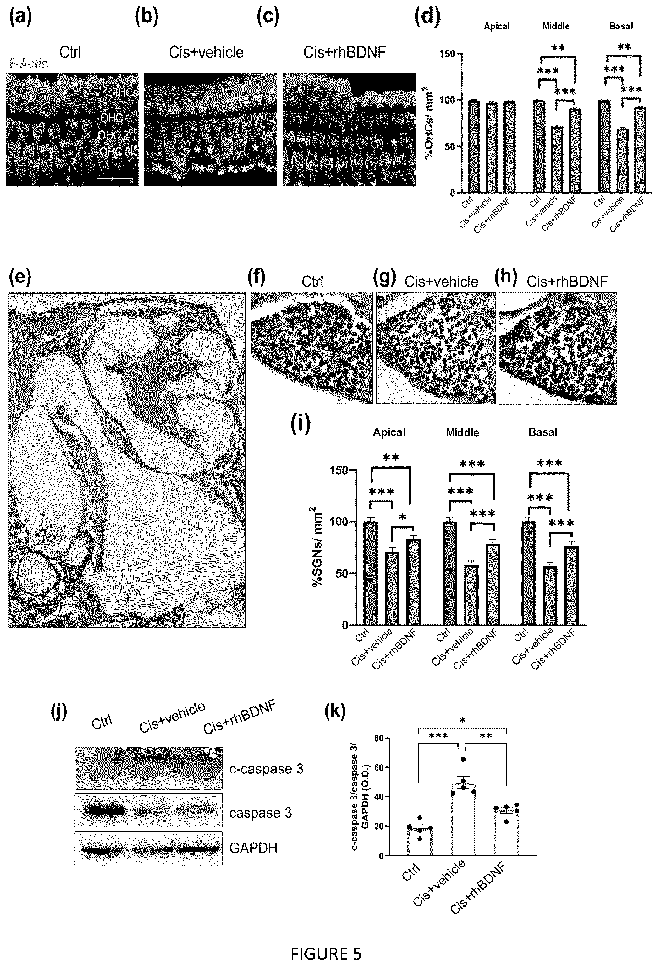

**Figure 5****:** Panels (a)-(c) show representative images of surface preparations of the organ of Corti showing F-actin distribution in the middle-basal cochlear turn. A normal cochlear organization, represented by one row of IHCs and three rows of OHCs, is observed in control animal (Ctrl, panel (a)). In Cis+vehicle group (panel (b)) a severe OHC loss was observed. White asterisks indicate missing OHCs (dark spots). rhBDNF treatment shows a protective effect against OHC death caused by cisplatin (panel (c)). Scale bar: 25 µm. (d): Cochleograms (means ± SEM) showing the percentage of OHC survival in all cochlear turns (n=5 rats for each group; one-way Anova, Middle turn: Ctrl vs Cis+vehicle, p< 0,0001; Ctrl vs Cis+rhBDNF, p=0,0015, Cis+vehicle vs Cis+rhBDNF p= 0,0002. Basal turn: Ctrl vs Cis+vehicle, p<0,0001; Ctrl vs Cis+rhBDNF, p=0013, Cis+vehicle vs Cis+rhBDNF p< 0,0001). Panels (e)-(h) show representative H&E images of SGN cryo-sections. Panel (e): representative image of a cochlear cross-section stained with H&E (Evos; 10x) of a Ctrl group animal. Panel (f): normal morphology of SGNs in a Ctrl group animal (Evos; 20x). Panel (g): SGN loss after cisplatin treatment in a Cis+vehicle group animal. Panel (h): recovery of neuron viability after rhBDNF treatment in a Cis+rhBDNF group animal. Panel (i) shows a histogram representing SGN viability (means ± SEM) in Ctrl, Cis+vehicle, Cis+rhBDNF group animals (n=5 rats for each group; one-way Anova, Apical turn: Ctrl vs Cis+vehicle, p< 0,0001; Ctrl vs Cis+rhBDNF, p=0,0064, Cis+vehicle vs Cis+rhBDNF p= 0,047. Middle turn: Ctrl vs Cis+vehicle, p< 0,0001; Ctrl vs Cis+rhBDNF, p=0,0001, Cis+vehicle vs Cis+rhBDNF p= 0,0004. Basal turn: Ctrl vs Cis+vehicle, p <0,0001; Ctrl vs Cis+rhBDNF, p <0,0001, Cis+vehicle vs Cis+rhBDNF p= 0,0002). Asterisks are referred to significant differences between groups (*p < 0.05; **p < 0.01; ***p < 0.001). Panel (j) shows a representative western blot image showing cleaved-caspase-3 (c-caspase 3) protein levels in Ctrl, Cis+vehicle, Cis+rhBDNF groups. Panel (k) shows an histogram representing the results of densitometric analyses of the western blot band of c-capsase-3 in Ctrl, Cis+vehicle, Cis+rhBDNF group animals, measured as described in Example (n = 5 rats for each group; one-way Anova, p<0,0001 Ctrl vs Cis + vehicle; p =0,045 Ctrl vs Cis + rhBDNF; p= 0,0023 Cis+vehicle vs. Cis + rhBDNF) normalized to the corresponding total protein levels (caspase 3 and GAPDH). Data are expressed as mean ± SEM. Asterisks are referred to significant differences between groups (*p < 0.05; **p < 0.01 ;***p < 0.001).

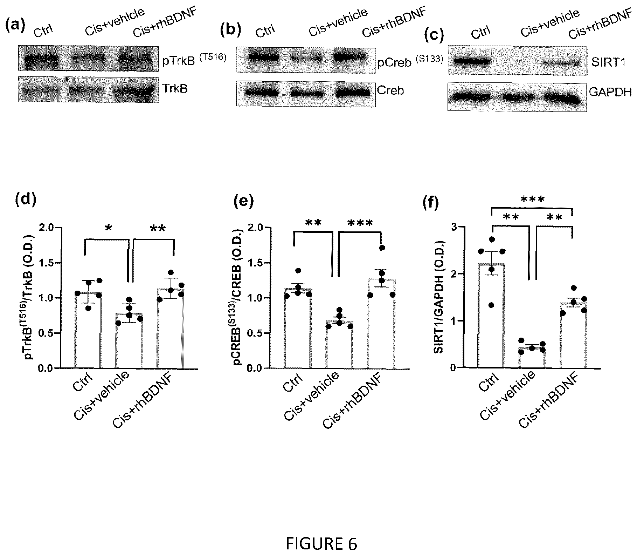

**Figure 6****:** Panels (a)-(c) show representative western blot images showing pTrkB (panel (a)), pCREB (panel (b)) and SIRT1 (panel (c)) expression in Ctrl, Cis+vehicle and Cis +rhBDNF animal groups, as described in Example 2 . Panels (d)-(f) show histograms (means ± SEM) representing densitometric analyses for pTrkB (n = 5 rats for each group; one-way Anova, p = 0.0238 Ctrl vs Cis + vehicle; p = 0.0069 Cis+vehicle vs Cis + rhBDNF), for pCREB (n = 5 rats for each group; one-way Anova, p = 0.0086 Ctrl vs Cis + vehicle; p = 0.0009 Cis+vehicle vs. Cis + rhBDNF) and Sirt1 (n = 5 rats for each group; one-way Anova, p<0,0001 Ctrl vs Cis + vehicle; p = 0.008 Ctrl vs Cis+rhBDNF; P=0,0029 Cis+vehicle vs. Cis + rhBDNF) normalized to the corresponding total protein levels (TrkB and CREB) or GAPDH. Data are expressed as mean ± SEM. Asterisks are referred to significant differences between groups (*p < 0.05; **p < 0.01; ***p < 0.001).

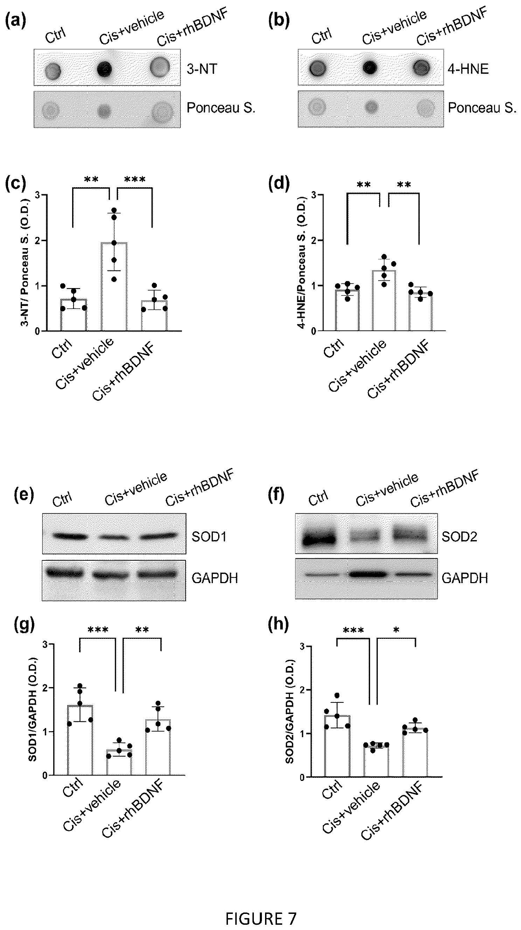

**Figure 7****.** Panels (a)-(b) show representative dot blot images showing expression of 3-NT (panel(a)) and 4-HNE (panel (b)) in Ctrl, Cis+vehicle and Cis+rhBDNF animal groups, as described in Example 2, measured as described in Example 7. Panels (c)-(d) show histograms (means ± SEM) representing results of densitometric analyses normalized to total protein levels detected by Ponceau staining (n = 5 rats for each group; one-way Anova. 3-NT: p = 0.0012 Ctrl vs Cis + vehicle; p = 0.001 Cis+vehicle vs Cis + rhBDNF; 4-HNE: p = 0.0047 Ctrl vs Cis + vehicle; p = 0.0017 Cis+vehicle vs Cis + rhBDNF). Panels (e)-(f): show representative western blot images showing expression of SOD1 (panel (e)) and SOD2 (panel (f)) in Ctrl, Cis+vehicle and Cis+rhBDNF animal groups, as described in Example 2, measured as described in Example 7. Panels (g)-(h) show histograms (means ± SEM) representing the results of densitometric analyses normalized to total protein levels (GAPDH) (n = 5 rats for each group; one-way Anova. SOD1: p = 0.0003 Ctrl vs Cis + vehicle; p = 0.0067 Cis+vehicle vs Cis + rhBDNF; SOD2: p = 0.0002 Ctrl vs Cis + vehicle; p = 0.0133 Cis+vehicle vs Cis + rhBDNF). Asterisks are referred to significant differences between groups (*p < 0.05; ** p < 0.01).

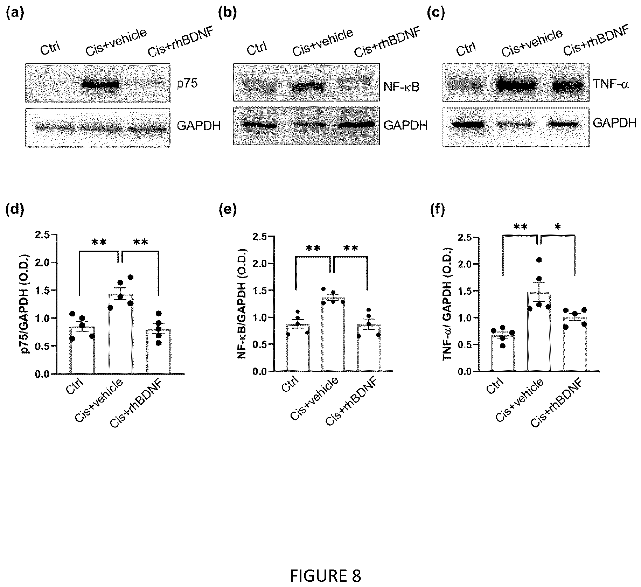

**Figure 8****:** Panels (a)-(c) show representative western blot images showing p75, NF-kB and TNF-a expression Ctrl, Cis+vehicle and Cis+rhBDNF animal groups, as described in Example 2, measured as described in Example 8. Panels (d)-(f) show histograms (means ± SEM) representing the results of densitometric analyses (n = 5 rats for each group; one-way Anova. p75: p = 0.0026 Ctrl vs Cis + vehicle; p = 0.0015 Cis+vehicle vs Cis + rhBDNF; NF-κB: p = 0.0024 Ctrl vs Cis + vehicle; p = 0.0023 Cis+vehicle vs Cis + rhBDNF; TNF-α: p = 0.0011 Ctrl vs Cis + vehicle; p = 0.0425 Cis+vehicle vs Cis + rhBDNF) normalized to the total protein levels (GAPDH). Asterisks are referred to significant differences between groups (*p < 0.05; ** p < 0.01).

### DETAILED DESCRIPTION OF THE INVENTION

A first object of the invention is Brain-Derived Neurotrophic Factor (BDNF) for use in the prevention of a hearing loss induced by a platinum-based chemotherapy drug in a subject, wherein said BDNF is administered intratympanically to said subject.

As used herein, the term "intratympanical" or "intratympanic" refers to accessing or entering into the middle ear. Therefore, intratympanical administration refers to the administration into the middle ear. Intratympanical administration may be performed through the eardrum, but may also be done in a different way, for example through the Eustachian tube.

It has been demonstrated by the present inventors in the experimental section that the intratympanic administration of BDNF temporally close to the administration of cisplatin is surprisingly able to completely counteract the toxic effect of cisplatin on the various structures of the cochlea, thus preventing the onset of a hearing loss in subjects receiving platinum-based chemotherapy drugs.

According to a preferred embodiment of the present invention, the prevention activity of BDNF consists in reducing the likelihood to have a hearing loss induced by said platinum-based chemotherapeutic in said subject.

Accordingly, said BDNF is preferably for use in the reduction of the likelihood to have a hearing loss induced by a platinum-based chemotherapy drug in a subject, wherein said BDNF is administered intratympanically to said subject.

Preferably, said BDNF reduces of at least 40%, preferably of at least 50%, more preferably of at least 60%, even more preferably of at least 70%, even more preferably of at least 80% the likelihood for the subject to have a hearing loss induced by said platinum-based chemotherapy drug in said subject.

According to another equally preferred embodiment of the present invention, the prevention activity of BDNF consists in preventing the onset of a hearing loss induced by a platinum-based chemotherapy drug in said subject.

Accordingly, said BDNF is preferably for use in the prevention of the onset of a hearing loss induced by a platinum-based chemotherapy drug in a subject, wherein said BDNF is administered intratympanically to said subject. In this specific context, prevention should be interpreted as avoidance of the event occurring.

Preferably, said BDNF for use according to the invention, is administered to said subject as sole preventive therapy of hearing loss induced by a platinum-based chemotherapy drug. Preferably, said BDNF for use according to the invention, is not administered in combination with other therapeutic agents aimed at preventing hearing loss induced by platinum-based chemotherapy drug in said subject.

Preferably, said subject is a human subject. More preferably, said subject is a pediatric human subject. Preferably, said subject has been diagnosed with a solid cancer and is receiving a therapy based on platinum-based chemotherapy drug.

Preferably, the platinum-based chemotherapy drug is selected from cisplatin, carboplatin, oxaliplatin, nedaplatin, triplatin tetranitrate, phenanthriplatin, picoplatin, and satraplatin, more preferably from cisplatin, carboplatin, oxaliplatin and nedaplatin, even more preferably it is cisplatin.

Preferably, said BDNF is human BDNF, more preferably it is recombinant human BDNF (rhBDNF).

Preferably, said BDNF has a purity higher than 70%, more preferably higher than 80%, higher than 90%, higher than 95%, higher than 98% or higher than 99%.

Preferably, said BDNF for use according to the invention is administered to said subject prior to, concurrently with, or after the administration of the platinum-based chemotherapy drug, at a time point between 6 hours prior to and 6 hours after the administration of said platinum-based chemotherapy drug, more preferably at a time point between 2 hours prior to and 4 hours after the administration of said platinum-based chemotherapy drug. Preferably, said BDNF is administered to said subject 1 hour after the administration of said platinum-based chemotherapy drug.

Preferably, said BDNF for use according to the invention is administered in a pharmaceutical acceptable composition for intratympanic administration, preferably, as described below.

A further object of the present invention is a pharmaceutical composition for intratympanic administration comprising BDNF for use as described above and one or more pharmaceutically acceptable excipients.

Preferably, said BDNF for use according to the invention is administered in said pharmaceutical composition at a concentration of between 1 mg/ml and 15 mg/ml, more preferably between 3 mg/ml and 10 mg/ml, even more preferably between 4 mg/ml and 7 mg/ml, even more preferably of 7 mg/ml.

Preferably, said pharmaceutical composition comprises BDNF, preferably at the concentration indicated above, and at least one pharmaceutically acceptable excipient, more preferably selected from solvents, viscosity enhancers, thickening agents, mucoadhesive agents, buffers, antioxidants, surfactants, preservatives, and penetration enhancers.

More preferably, said pharmaceutical composition comprises an BDNF, preferably at the concentration indicated above, and at least one viscosity enhancer, at least one poloxamer, and an aqueous solvent, according to the concentrations disclosed above. Preferably, said pharmaceutical composition is in form of a thermoreversible gel formulation.

The advantage to use a thermoreversible gel formulation is that the diffusion of rhBDNF is prevented, and the solidification of the gel allows a long-term contact with the tympanic membrane, ensuring a continuous and constant release of rhBDNF over time.

Preferably, the thermoreversible gel formulation according to the present invention comprises at least one viscosity enhancer and at least one poloxamer, as pharmaceutically acceptable excipients.

Preferably, said viscosity enhancer is hydroxypropyl methylcellulose, more preferably hydroxypropyl methylcellulose, and it is present in said thermoreversible gel formulation at a concentration comprised between 0.05%(w/v) and 0.2%(w/v), preferably 0.1% (w/v). Preferably, said at least one poloxamer is Poloxamer 188 (Kolliphor P188) and/or Poloxamer 407 (Kolliphor P407), preferably a combination of Poloxamer 188 (Kolliphor P188) and Poloxamer 407 (Kolliphor P407).

Preferably, said Poloxamer 188 (Kolliphor P188) is present in said thermoreversible gel formulation at a concentration between 2%(w/v) and 4%(w/v), preferably 3%(w/v). Preferably, said Poloxamer 407 (Kolliphor P407) is present in said thermoreversible gel formulation at a concentration between 20%(w/v) and 25%(w/v), preferably 23%(w/v). Preferably, the thermoreversible gel formulation comprises at hydroxypropyl methylcellulose, more preferably hydroxypropyl methylcellulose, Poloxamer 188 (Kolliphor P188), Poloxamer 407 (Kolliphor P407), as pharmaceutically acceptable excipients.

The term "Kolliphor P188", as used herein, refers to Poloxamer 188, a block copolymer that is a synthetic copolymer of ethylene oxide and propylene oxide represented by the following chemical structure: 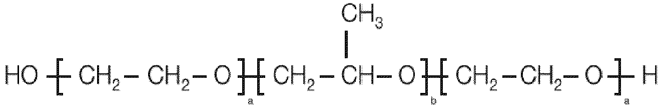 wherein a and b blocks have the following values:

| **Kolliphor^{®}** | **Poloxamer** | **a** | **b** |
|---|---|---|---|
| P 188 | 188 | 80 | 27 |

The term "Kolliphor P 407", as used herein, refers to Poloxamer 407, a block copolymer that is a synthetic copolymer of ethylene oxide and propylene oxide represented by the following chemical structure: 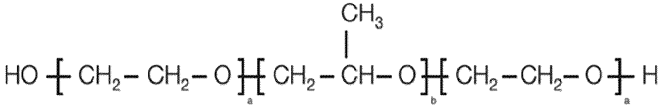 where the a and b blocks have the following values:

| **Kolliphor^{®}** | **Poloxamer** | **a** | **b** |
|---|---|---|---|
| P407 | 407 | 101 | 56 |

More preferably, said thermoreversible gel formulation comprises, preferably consists of, BDNF, preferably at the concentrations indicated above, hydroxypropyl methylcellulose (HPMC), Poloxamer 188, and Poloxamer 407, according to the concentrations disclosed above, and an aqueous solvent.

Preferably, said aqueous solvent is water and/or phosphate buffer.

Preferably, said thermoreversible gel formulation comprises, preferably consist of, the following excipients in an aqueous solvent:
a) between 0.05 and 0.2%, preferably 0.1% of hydroxypropyl methylcellulose;
b) between 2 and 4%, preferably 3%, of Poloxamer 188 (Kolliphor P188);
c) between 20 and 25%, preferably 23%, of Poloxamer 407 (Kolliphor P407).

More preferably, said thermoreversible gel formulation comprises the following excipients in an aqueous solvent:
a) 0.1% of hydroxypropyl methylcellulose;
b) 3% of Poloxamer 188 (Kolliphor P188);
c) 23% of Poloxamer 407 (Kolliphor P407).

Preferably, said aqueous solvent is water and/or phosphate buffer.

Preferably, said pharmaceutical composition is for use in the prevention of a hearing loss induced by a platinum-based chemotherapy drug in a subject, as disclosed above. Preferably, said pharmaceutical composition is administered intratympanically to said subject.

Preferably, said pharmaceutical composition is in form of a thermoreversible gel formulation, according to the above.

Preferably, the presence of said hearing loss is identified by means of an audiometry test. Preferably, no hearing loss is considered as present in subjects that in an audiometry test detect sounds below 25 dB at frequencies of 250-8,000 Hz.

Preferably, said prevention of a hearing loss is determined by comparing the hearing function of the patient after administration of the platinum-based chemotherapy drug to the baseline hearing function of the subject prior to administration of the platinum-based chemotherapy drug.

The invention will be further described in the following examples, which do not limit the scope of the invention defined in the claims.

### EXPERIMENTAL SECTION

### Example 1 - Formulation preparation

A thermoreversible gel vehicle was prepared according to the following procedure.

Hydroxypropyl cellulose 4K was added in 100 mL of water at a concentration of 0.1% (w/v), and complete solubilization was obtained under magnetic stirring. Then, 3% (w/v) Kolliphor P188 and 20% (w/v) Kolliphor P407 were added to the solution. After the addition of Kolliphor(s), the formulation was placed at +2/+8 °C under gently stirring for about 24h to obtain a clear colorless solution.

The thus-obtained gel vehicle formulation consisted of:
20% (w/v) Kolliphor P407,
3% (w/v) Kolliphor P188,
0.1% (w/v) HPMC 4K, and
WFI (water for injection).

The cold formulation was filtered with a 0.2 µm filter.

The obtained formulation is a thermoreversible gel, which is liquid at +2/+8 °C and turns to semisolid state (gel state) at 35±0.5 °C.

Two formulations, containing different concentrations of rhBDNF were prepared in the above vehicle formulation:
- BDNF at 5 µg/µL:
   375 µg of rhBDNF were added in 0.075 mL of the above gel vehicle; then, phosphate buffer was added to reach 0.35 mL final volume per vial. The sample was then freeze-dried. The freeze-dried sample was reconstituted with 0.075 mL of reconstitution solvent (Water for injection containing Kolliphor P407 3% w/v) to a obtain thermoreversible gel containing rhBDNF at a concentration of 5 µg/µL.
- BDNF at 3 µg/µL:
   225 µg of rhBDNF were added in 0.075 mL of the above gel vehicle; then, phosphate buffer was added to reach 0.35 mL final volume per vial. The sample was then freeze-dried. The freeze-dried sample was reconstituted with 0.075 mL of reconstitution solvent (Water for injection containing Kolliphor P407 3% w/v) to a obtain thermoreversible gel containing rhBDNF at a concentration of 3 µg/µL.

### Example 2- In vivo experiment

The ability of rhBDNF to prevent cisplatin-induced hearing loss was tested in an in-vivo rat model according to the procedure outlined in Figure 1(a).

In details, two-month-old male Wistar rats (weighing between 200 and 250 g) from Catholic University Laboratories were used in this study. Animal procedures were conducted in compliance with the Laboratory of Animal Care and Use Committee of the Catholic University, School of Medicine of Rome and were approved by the Italian Department of Health (Ministero della Salute, Prot. 1F295.111.EXT.77).

All efforts were made to reduce the number of animals and to minimize their suffering in accordance with the European Community Council Directive of 24 November 1986(86/609/EEC). Animals were housed two per cage into Catholic University Animal facility at a controlled temperature (22-23 °C) and constant humidity (60-75%), under a 12 h light/dark cycle, with food (Mucedola 4RF21, Italy) and water ad libitum.

All the experiments were performed on a total of 80 animals, randomized and assigned to 6 experimental groups, as follows:
(1) control group (untreated animals) ("Ctrl", n= 23);
(2) control animals treated with vehicle in the left ears ("Vehicle", n=5);
(3) cisplatin-treated group ("Cis" n= 10);
(4) animals treated with cisplatin and with vehicle in right ears ("Cis+vehicle", n= 7);
(5) animals treated with cisplatin, vehicle in the left ears and rhBDNF (3 µg/µl) in the right ears ("Cis+rhBDNF (3 µg/µl)", n= 12);
(6) animals treated with cisplatin, vehicle in the left ears and rhBDNF (5 µg/µl) in the right ears ("Cis + rhBDNF (5 µg/µl)" n= 23).

Animals of groups 4-6 were treated with cisplatin at time 0 as follows. Cisplatin (cis-Diammineplatinum(II) dichloride; Cat. No. P4394, Sigma-Aldrich, St. Louis, MO, USA), was dissolved freshly in sterile saline (1 mg/ml) and protected by light. The solution was heated for about 20 minutes to facilitate cisplatin dissolution. Animals were deeply anesthetized (Ketamine 87,5 mg/Kg + Xylazine 12,5 mg/Kg) and a single cisplatin dose of 12 mg/Kg was delivered intraperitoneally (i.p.) at a rate of 8 ml/h with an infusion pump (Axon Instruments, Foster City, CA, USA) over about 30 minutes. The dosage of 12 mg/kg was selected as the optimal dose, because it achieved the desired level of ototoxicity while maintaining an acceptable mortality rate (Waissbluth et al., Eur Arch Otorhinolaryngol 2012, 270, 1597-1605; Chen et al., Int J Nanomedicine 2019, 14, 4211-4227). Following treatment, rats were hydrated daily with a saline solution (subcutaneous injection, 15 ml) to reduce other systemic side effects of cisplatin.

Animals of groups 2 and 4-6 were treated with rhBDNF 1 hour after cisplatin treatment. In details, animals were deeply anesthetized (Ketamine 87,5 mg/Kg + Xylazine 12,5 mg/Kg) and a single intratympanic injection was performed one hour after cisplatin administration, using a rhBDNF formulation described in Example 1, in the right ears of animals of groups 5 (rhBDNF 3 µg/µl formulation) and 6 (rhBDNF 5 µg/µl formulation) and a vehicle formulation described in Example 1 in the right ears of animals of group 4 and in the left ears of animals of group 2, 5 and 6. To perform intratympanic injection, the round window (RW) and the niche of RW were localized with the help of a surgical microscope (Zeiss operating microscope, Germany). Injection was performed using a 100 µl luer lock Hamilton syringe using an operating microscope. A small perforation of the tympanic membrane was performed anterior to the malleus to equalize middle ear pressure (Paciello et al., Frontiers in Pharmacology 2018, 9). A second perforation was made in a postero-inferior membrane region, through which 30 µl of either the rhBDNF formulation or vehicle were injected at a constant rate (2 µl/s) into the RW niche area. Following the administration, the animals were maintained for at least 10 min with the treated ear facing upward to ensure test molecule delivery and contact with the RW. The same procedure will be then repeated in the left contralateral ear injecting the vehicle solution.

All procedures were conducted with aseptic techniques under aseptic conditions.

To detect the best rhBDNF dosage, a dose-response curve was obtained from 6 animals per group by using the two concentrations of rhBDNF tested (3 and 5 µg/µl).

Auditory Brainstem Responses (ABR) analyses were performed at day 3 and 7 after cisplatin treatment to assess auditive function, as described in Example 4 below.

The animals were sacrificed at day 7 and cochlear tissue collected for the different analysis described in Examples below.

### - Data and statistical analysis

Statistical analysis was performed using Prism 8.0 (GraphPad Software, San Diego, CA, USA). The data were presented as mean ± standard error of the mean (SEM). To compare two groups, the two-tailed Student's t-test was utilized. For multiple group comparisons, analysis of variance (ANOVA) was employed, followed by the Bonferroni correction to compare means. A significance level of p < 0.05 was considered statistically significant.

### Example 3 - TrkB expression and localization in cochlear structures

Immunofluorescence experiments were carried out on the tissue collected from animals of the control groups of Example 2 to identify the presence of BDNF high-affinity receptor, TrkB, in the cochlea.

In details, immunofluorescence analysis was performed on formaldehyde-fixed cochlear cryo-sections and whole-mount cochlear preparations. Cochleae were extracted and fixed in 4% paraformaldehyde in PBS at a temperature of 4°C and pH 7.5. To perform immunofluorescence on cochlear longitudinal sections, samples were decalcified for 15 days using 10% EDTA, followed by a 48-hour incubation in 30% sucrose Then, cochleae were embedded in OCT (Killik, Bio-optica, Milan, Italy) and cryosectioned at a thickness of 12 µm using the Cryostat (CM 1950 from SLEE medical GmbH, Mainz, Germany). To perform immunofluorescence on stria vascularis whole mount, stria vascularis was isolated after cochlear dissection as described previously (Paciello et al Front Cell Dev Biol 2022 10, 950837). Cochlear sections and stria vascularis whole mount were incubated in a blocking solution containing 1% BSA, 0.5% Triton X-100 and 10% normal goat serum in PBS for 1h at room temperature. Samples were then incubated overnight at 4°C with a solution containing TrkB primary antibody (ab18987, Abcam. Samples were washed in PBS and incubated at room temperature for 2 hours, light protected, in Alexa Fluor-labeled anti-rabbit secondary antibody (Alexa Fluor 546, Cat. No. A110040) diluted 1:400 in 0.1 M PBS. After two more washes in PBS, samples were counterstained with DAPI (1:1000 diluted in PBS) for 20 minutes in the dark at room temperature and then mounted onto non polarized glass slide with a mounting medium (ProLongTM Gold Antifade Mountant). Images were captured using a confocal laser scanning microscope. Fluorescent images were obtained with a confocal laser microscope (Nikon Ti-E) with a 20× or 60× objective lens.

For all immunofluorescence procedures described herein, to ensure accurate analysis, control experiments were conducted by excluding the primary antibody during tissue processing, with tissues selected randomly from the experimental groups. In cochlear samples, no staining was observed, indicating the absence of spontaneous fluorescence or non-specific antibody binding (data not shown). Throughout the procedures, tissues from all groups were consistently processed together to minimize variability related to antibody penetration, incubation time, post-sectioning age, and tissue condition.

As shown in Figure 1b), the images obtained demonstrate a specific localization of TrkB in SGNs (right image), in the outer and inner hair cells (OHCs and IHCs) of the Organ of Corti and in primary afferent fibers (Fibers) (left image).

### Example 4 - rhBDNF administration prevents hearing loss induced by cisplatin treatment

To assess hearing loss induced by cisplatin treatment and the protective effect of rhBDNF administration on hearing function, the inventors performed an audiological test by measuring Auditory Brainstem Responses (ABRs), one day before cisplatin treatment (baseline) and 3-days (D3) and 7-days (D7) after cisplatin administration, as described in Example 2 (see Figure 1a).

In details, in all animals, ABRs were evaluated bilaterally at different frequencies (6, 12, 16, 20, 24 and 32 kHz). Rats were mildly anesthetized (ketamine, 35 mg/kg and medetomidine-domitor, 0.25 mg/kg) and placed in the anechoic room. As previously reported (Paciello et al, Elife 202,110, e70908), three stainless steel recording electrodes were subcutaneously inserted into the right mastoid (active), vertex (reference) and left mastoid (ground). Auditory stimulus generation and auditory brainstem response (ABR) recording were conducted using a PC-controlled TDT System 3 (Tucker-Davis Technologies, Alachua, FL, United States) data acquisition system, which incorporated real-time digital signal processing. Monaural presentation of tone bursts, ranging from 6 to 32 kHz (1 ms rise/fall time, 10 ms total duration, 20/s repetition rate), was performed in an open field setting. The recorded responses were filtered (0.3-3 kHz), digitized, and averaged across 500 discrete samples for each frequency-sound level combination. Threshold values were determined as the minimum stimulus level (in dB) at which a consistent waveform-based onset could be observed, indicating repeatability.

To investigate the neural transmission time of the cochlear nucleus, the latency of Wave II was also examined. The latency of ABR components was defined as the time from the computer triggering the earphone to the waveform positive peak, including a 0.3 ms acoustic transit time between the earphone and the animal pinnae (Church et al., Clinical and Experimental Research 2011, 36, 83-96; Fetoni et al., J Neurosci 2013, 33, 4011-4023).

Figures 2 report graphs (means±SEM) showing the mean auditory threshold shift in the Cis+vehicle (Fig 2a, 2b, 2d, 2e), Cis+rhBDNF (3 µg/µl) (Fig. 2a, 2d), Cis+rhBDNF (5 µg/µl) (Fig 2b, 2e), Ctrl (Fig 2c and 2f) and Vehicle (Fig 2c and 2f) groups, as described in Example 2, measured at 3 days (D3) (Fig 2a, 2b and 2c) and 7 days (D7) (Fig 2d, 2e and 2f) after cisplatin injection.

The results show a significant increase of the threshold shift in animals of the Cis+vehicle group compared to controls (Ctrl and Vehicle), while no significant differences in ABR threshold shifts were observed between controls and cisplatin-treated animals administered with the thermoreversible gel containing 3 µg/µl or 5 µg/µl BDNF according to the present invention ("Cis+rhBDNF").

These results clearly demonstrate that the administration of rhBDNF, shortly after administration of cisplatin, completely inhibits the onset of cisplatin-induced hearing loss. Furthermore, the results show that the best auditory protection was obtained with a dosage of 5 µg/µl BDNF at all time points analyzed.

Therefore, all the immunofluorescence and molecular analyses carried out in the following Examples were performed on animals treated with the highest protective dosage (5 µg/µL). To better characterize functional damage, the inventors also evaluated the neural transmission time by studying the latency of ABR wave II (P2) at 24 kHz, (the frequency in which the best protective function of rhBDNF was observed).

The results are shown in Figure 3(a) and demonstrate a significant increase of latency in Cis+vehicle group compared to control (Ctrl) group, while no significant differences were observed between Cis+rhBDNF and the control (Ctrl) groups.

The above results unexpectedly highlight the complete preventive activity of rhBDNF on hearing loss due to cisplatin treatment, allowing the hearing function of cisplatin-treated animals to be maintained at the same level as the hearing function of control animals.

### Example 5 - Neuroprotective effect of rhBDNF administration against cisplatin-induced synaptopathy and afferent fiber degeneration.

To study synapses between inner hair cells (IHCs) and neuronal afferent fibers, an immunofluorescence analysis on surface preparations of the organ of Corti was carried out. 6 cochleae collected from 6 rats were quickly removed and fixed with 4% paraformaldehyde. After tissue dissection, the basilar membrane was isolated, and specimens were incubated with a blocking solution containing 1% BSA, 0.5% Triton X-100 and 10% normal goat serum in PBS for 1h at room temperature. Then, samples were incubated overnight at 4 °C with a solution containing specific primary antibodies: mouse anti-transcriptional regulator C-terminal binding protein 2 (CtBP2-lgG1, 1:200; BD Transduction Laboratories) and mouse anti-GluA2 (IgG2a, GluR2/GluA2; 1:500; Millipore). Samples were washed in PBS and incubated at room temperature for 2 h, light protected, with labelled-conjugated donkey anti-rabbit and/or anti-mouse secondary antibodies (Alexa Fluor 488 or 546, IgG, Thermo Fisher) diluted 1:400 in 0.1 M PBS. After two more washes in PBS, samples were counterstained with DAPI (1 :1000 diluted in PBS) for 20 min in the dark at room temperature and then mounted onto non polarized glass slide with a mounting medium (ProLongTM Gold Antifade Mountant).

A confocal microscope system (NikonTi-E, Confocal Head A1 MP, Tokyo, Japan) was used to capture images of the immune-labeled specimens using 60× objective lens. A paired synapse was identified as one showing co-localization of CtBP2 and GluA2 positive puncta, and the number of synaptic ribbons was then quantified using a compressed z-stack of each image through image processing software (NIH imaged 1.43u, Image Processing and Analysis in Java). The total number of ribbons was divided by the number of IHCs to estimate the ribbon/IHC ratio.

Moreover, a quantification of total OHC and IHC neuronal afferent fibers was carried out. Dissected cochlear samples containing sensory-neuronal epithelium were incubated for 1 hour with a blocking solution, described in the previous section, and then incubated overnight at + 4 °C with a solution composed by the following primary antibodies: NF200 (N4142, Sigma-Aldrich), All specimens were incubated at RT for 1 hours in labelled conjugated goat anti-rabbit (Alexa Fluor 546 IgG) 1:400 in 0.1 M PBS. Afterward, the samples incubated in DAPI solution (1:500 in 0.1 M PBS) for 20 min in the dark at room temperature and then mounted onto non polarized glass slide with a mounting medium (ProLongTM Gold Antifade Mountant, Cat. No. P36930). Images were captured using a confocal laser scanning microscope (Nikon Ti-E) with a 20× or 60× objective lens. The analysis of number and fiber thickness was performed using NIH imaged 1.43u, Image Processing and Analysis in Java.

The results of the immunofluorescence analysis revealed a decrease in the number of synaptic ribbons in the Cis+vehicle group compared to the control group, as demonstrated by histograms representing the count of paired synaptic ribbons (Figure 3b). Representative images of surface preparations of the organ of Corti, stained with anti-CtBP2 (red fluorescence for labelling synaptic ribbons) and anti-GluA2 (green fluorescence for visualizing post-synaptic puncta), and double-stained with DAPI, were obtained.

In control specimens, it was clearly identified juxtaposed pre-synaptic ribbons and post-synaptic receptors (Figure 3c, c1). However, in Cis+vehicle group, the number of paired ribbon synapses significantly decreased by approximately 70% compared to the control group (Figure 3b, d, d1). After administering rhBDNF, an increase of about 35% of synaptic contacts compared to the Cis+vehicle group (Figure 3b, e, e1) was observed.

Thus, the results obtained show that functional impairment induced by cisplatin is associated with cochlear synaptic damage, and that this can be prevented by treatment with rhBDNF.

To better understand the effects of cisplatin and rhBDNF on afferent fibers, an immunofluorescence analysis, targeting Neurofilament 200 (NF200) (a well-established marker of neurofilaments) was carried out.

A count of the number of fibers in a randomly selected 50 µm segment was also carried out, and fiber thickness was evaluated.

The analysis revealed a decrease of primary afferent fibers within OHCs and SGNs (Figure 4a, 4b) and within IHCs and SGNs (Figure 4c, 4d) in animals exposed to cisplatin, as compared to Ctrl animals; however, following the administration of rhBDNF, a restoration in fiber density was observed.

Moreover, the results demonstrated a significant decrease in both the number and thickness of fibers following cisplatin treatment, while a significant increase of both parameters was observed after rhBDNF administration.

Overall, these results showed that rhBDNF administration protected against cisplatin-induced synaptopathy by increasing synaptic contacts, as well as restoring afferent fiber damage.

### Example 6 - Protective effects of rhBDNF against cisplatin-induced cochlear cell death

The protective effect of rhBDNF on hair cell induced by cisplatin, OHC viability in organ of Corti surface preparations was analyzed by F-Actin staining.

In details, cochleae from 6 animals/group were quickly removed and fixed with 4% paraformaldehyde. After tissue dissection, the basilar membrane was isolated and F-actin staining was performed by incubating samples with ActinGreen 488 Ready Probes Reagent (ThermoFisher, Cat. No. R37110). After washing with PBS, the samples were mounted onto glass slides with a mounting medium (FluorSaveTM Reagent, Merk, Cat. No. 345789) and analyzed using a confocal microscope (NikonTi-E) equipped with a 40× objective lens. Figure 5a-c show OHC viability in organ of Corti surface preparations analyzed by F-Actin staining. As can be seen, in Ctrl rats, OHC surfaces exhibited a well-organized arrangement of three rows of OHCs (Figure 5a). Cisplatin treatment resulted in a substantial loss of OHCs, characterized by dark spots (indicated by white asterisks, Figure 5b), which was measured as of about 40% in the middle turns and in the basal turn, compared to the Ctrl group (as shown in the histograms, Figure 5d). Notably, treatment with rhBDNF demonstrated a significant protective effect (Figure 5c), reducing OHC loss to about 10% (Figure 5d).

The protective effect of rhBDNF on spiral ganglion neuron (SGN) death was evaluated by Hematoxylin and Eosin (H&E) staining. Cochleae from 6 animals/group were quickly removed and fixed with 4% paraformaldehyde in PBS at 4 °C, at pH 7.5 for almost 24 hours, then decalcified for 15 days in 10% EDTA, followed by a 48-hour incubation in 30% sucrose. The tissues were then embedded in OCT (Killik, Bio-optica, Milan, Italy) and cryosectioned at a thickness of 12 µm using the Cryostat (CM 1950 from SLEE medical GmbH, Mainz, Germany). SGN viability was examined using the H&E staining kit protocol (H&E Staining Kit, ab245880, abcam). The protocol involved a 5-min incubation of hematoxylin followed by a 2-3 min incubation of eosin. The stained samples were then mounted using Entellan^{®} (Cat. No. 107960, Merck, Germany). Subsequently, NIH imaged 1.43u (Image Processing and Analysis in Java) was used for neuronal count. Only neurons exhibiting a distinct round nucleus and uniform cytoplasm were included in the cell count, which was performed within a designated area of 100x100 µm. Cell viability was calculated by determining the percentage of surviving cells relative to the control group.

SGN viability examined by H&E staining performed on cochlear cryosections is shown in Figure 5e-h. In Ctrl group, the Rosenthal's canal was densely packed with SGNs (Figure 5f). After 7 days from the start of cisplatin treatment, a marked reduction of SGNs was observed in all cochlear turns (40% of SGN loss in the middle and basal turns and about 25% in the apical turn as represented in Figure 5i). After rhBDNF administration, an increase of SGN viability of about 20% in the middle and basal turns and of about 10% in the apical turn was observed, as shown by histograms representing the percentage of survival neurons (Figure 5i).

In addition, a Western Blot analysis of the expression of cleaved-caspase 3, which is considered a reliable marker for apoptotic cells (Crowley and Waterhouse, 2016. Detecting Cleaved Caspase-3 in Apoptotic Cells by Flow Cytometry. Cold Spring Harb Protoc), was carried out.

Protein lysates were obtained from 6 cochleae/animal group using the same protocol previously published (Fetoni et al., Free Radic Biol Med 2016, 101, 211-225; Paciello et al., Elife 2021, 10, e70908). Total proteins were extracted from cochlear tissues using ice-cold RIPA buffer (Pierce, Rockford, IL, USA, Cat. No. PI89900) containing protease and phosphatase inhibitors. Protein lysates (60 µg) were loaded onto 8 or 12% Tris-glycine polyacrylamide gels for electrophoretic separation. Precision Plus Protein Dual Color Standards (Biorad) were used as molecular mass standards.
factor

The results of Western Blot analysis showed that cisplatin treatment induced a significant increase of cleaved-caspase 3 expression, whereas rhBDNF treatment led to a reduction of its expression, confirming the protective effect of this neurotrophic factor against cochlear cell death induced by cisplatin (Figure 5j, k).

Overall, the results highlight the neuroprotective effect of rhBDNF administration against cisplatin-induced cochlear cell death and primary afferent fiber degeneration.

### Example 7 - rhBDNF otoprotective effects on cochlear oxidative stress and inflammation

It was examined whether local administration of rhBDNF could activate the TrkB/CREB pro-survival pathway by assessing the phosphorylated forms of TrkB receptor at tyrosine 516 (T516) and the transcription factor CREB at serine 133 (S133) by Western blot analysis. Notably, a significant reduction of the expression of both pTrkB and pCREB was observed after cisplatin treatment, compared to Ctrl group (Figure 6, panels (a), (b), (d) and (e)). Upon administering rhBDNF, an increase of pTrkB (Figure 6 panels (a), (d)) and pCREB (Figure 6, panel (b), (e)) was observed, indicating the activation of BDNF/TrkB signaling pathway.

Considering that CREB phosphorylation at S133 has been considered a redox-sensitive transcription factor, able to modulate the transcription of several antioxidant genes (Barlow et al., 2006; Bedogni et al., 2003; Sayed et al., 2020), SIRT1 expression in the experimental samples was analyzed.

The results revealed a marked reduction of SIRT1 levels following cisplatin exposure (Figure 6, panels (c), (f)). Interestingly, treatment with rhBDNF restored SIRT1 expression (Figure 6, panel (c), (f)), suggesting a potential involvement of the BDNF/TrkB signaling pathway in regulating endogenous antioxidant mechanisms.

To better understand the effect of rhBDNF treatment on cochlear redox status, it was also evaluated oxidative stress levels, by performing dot blot analysis, to detect protein tyrosine nitration (3-NT), a marker of nitro-oxidative stress, and 4-HNE expression, a marker of lipid peroxidation.

In details, in the dot blot procedure, 5 µl of lysates were carefully applied onto a nitrocellulose membrane pre-wetted with TBST. We ensured equal protein loading by staining the membrane with Ponceau S.

The membranes were incubated for 1h with blocking buffer containing 5% skim milk dissolved in TBST (Tris Buffered Saline and 0.1% Tween 20), and then incubated overnight at + 4°C with the following primary antibodies: 3-NT (Abcam, Cat. No. ab42789) and 4-HNE (Alpha Diagnostic International, Cat. No. #HNE11-S). After three 10-min rinses in TBST, the membranes were incubated for 1 h at RT with horseradish peroxidase (HRP)-conjugated mouse or rabbit secondary antibodies. Equal protein loading among individual lanes was confirmed by re-probing the membranes with an anti-GAPDH (ab8245, Abcam). The protein expression was evaluated and documented by using UVltec Cambridge Alliance. The experiments were performed in triplicate

The analyses demonstrated an increase of 3-NT and 4-HNE following cisplatin treatment (Figure 7a-d). However, the administration of rhBDNF significantly reduced the overexpression of both markers, as indicated by histograms representing densitometric analyses (Figure 7c, d).

To assess the alteration of the endogenous antioxidant system, it was analyzed the expression of superoxide dismutase 1 (SOD1) and manganese superoxide dismutase (SOD2), crucial scavengers of ROS that play essential roles in superoxide disproportionation and the rapid conversion of O2- to H2O2, by Western blot analysis. The results demonstrated a significant increase in both SOD1 and SOD2 levels following rhBDNF administration, highlighting the possible role of this neurotrophic factor in maintaining cellular redox balance (Figure 7, e-h).

Motivated by the presence of a redox unbalance in the cochlea induced by cisplatin and recognizing the frequent occurrence of inflammation as a consequence of both oxidative tissue damage and ototoxic insults, the inventors investigated by western blot analysis (carried out according to the above-mentioned protocol) the expression of cochlear inflammatory markers (Figure 8).

Specifically, the expression levels of p75 receptor (Figure 8a, d), NF-κB (Figure 8b, e) and TNF-α (Figure 8c, f) was evaluated.

The results show an upregulation of these markers following cisplatin treatment, accompanied by a notable decrease in their expression levels following the exogenous administration of rhBDNF (Figure 8).

The inventors demonstrated an increased TNF-α fluorescence signal in the marginal cell monolayer of the stria vascularis following cisplatin treatment, as evidenced by fluorescence intensity spectra, leading to a disruption in the structural integrity of marginal cells, as shown by a 2D reconstruction of confocal Z-stacks images, compared to the control group. Interestingly, treatment with rhBDNF reduced the expression of TNF-α, resulting in the preservation of structural integrity and stability of marginal cells.

The data relating to TNF-α fluorescence signal are not shown.

### Example 8 - rhBDNF treatment protects against vascular damage induced by cisplatin.

Finally, considering that cisplatin-induced cochlear damage involves not only oxidate and inflammatory insult but also vascular damage (Hamers et al., Audiology and Neurotology 2003, 8, 305-315), the effect of rhBDNF administration on angiogenic factors, such as Vascular Endothelial Growth Factor A (VEGFA), was evaluated by reconstruction of confocal Z-stacks images of TNFα(a-c) and VEGF-A (d-f) expression (red fluorescence) in stria vascularis whole-mounts stained with F-Actin (green fluorescence) and DAPI (blue fluorescence).

A homogeneous expression of VEGF-A in Ctrl animal, shown by fluorescence intensity spectrum, was observed. However, after cisplatin administration, a reduction in VEGF-A expression specifically in the damaged area of marginal cell monolayer was observed. Interestingly, after rhBDNF administration a high expression and localization of VEGF-A in marginal cells was observed.

Overall, the results revealed an increase of the expression and localization of VEGF-A in marginal cells, highlighting the potential of rhBDNF in promoting angiogenesis and preserving stria vascularis integrity in the cochlea.

## Claims

1. Brain-Derived Neurotrophic Factor (BDNF) for use in the prevention of a hearing loss induced by a platinum-based chemotherapy drug in a subject, wherein said BDNF is administered intratympanically to said subject.

2. Brain-Derived Neurotrophic Factor (BDNF) for use as claimed in claim 1, wherein said BDNF is administered to said subject prior to, concurrently with, or after the administration of the platinum-based chemotherapy drug, at a time point between 6 hours prior to and 6 hours after the administration of said platinum-based chemotherapy drug, preferably at a time point between 2 hours prior to and 4 hours after the administration of said platinum-based chemotherapy drug.

3. Brain-Derived Neurotrophic Factor (BDNF) for use as claimed in claim 2, wherein said BDNF is administered to said subject 1 hour after the administration of said platinum-based chemotherapy drug.

4. Brain-Derived Neurotrophic Factor (BDNF) for use as claimed in claims 1 to 3, wherein said BDNF is for use in the reduction of the likelihood to have a hearing loss induced by said platinum-based chemotherapeutic in said subject.

5. Brain-Derived Neurotrophic Factor (BDNF) for use as claimed in claim 1 to 4, wherein said BDNF reduces of at least 40%, preferably of at least 50%, more preferably of at least 60%, even more preferably of at least 70%, even more preferably of at least 80%, the likelihood to have a hearing loss induced by said platinum-based chemotherapy drug in said subject.

6. Brain-Derived Neurotrophic Factor (BDNF) for use as claimed in claims 1 to 3, wherein said BDNF is for use in the prevention of the onset of a hearing loss induced by a platinum-based chemotherapy drug in said subject.

7. Brain-Derived Neurotrophic Factor (BDNF) for use as claimed in claims 1 to 6, wherein said platinum-based chemotherapeutic is selected from cisplatin, carboplatin, oxaliplatin, nedaplatin, triplatin tetranitrate, phenanthriplatin, picoplatin, and satraplatin, preferably from cisplatin, carboplatin, oxaliplatin and nedaplatin, more preferably it is cisplatin.

8. Brain-Derived Neurotrophic Factor (BDNF) for use as claimed in claims 1 to 7, wherein said BDNF is administered in a pharmaceutical acceptable composition at a concentration of between 1 mg/ml and 15 mg/ml, more preferably between 3 mg/ml and 10 mg/ml, even more preferably between 4 mg/ml and 7 mg/ml, even more preferably of 7 mg/ml.

9. Brain-Derived Neurotrophic Factor (BDNF) for use as claimed in claims 1-8, wherein said BDNF is administered in a thermoreversible gel formulation.

10. Brain-Derived Neurotrophic Factor (BDNF) for use as claimed in claim 9, said thermoreversible gel formulation has the following composition:
a) between 0.05%(w/v) and 0.2%(w/v), preferably 0.1%(w/v) of hydroxypropyl methylcellulose;
b) between 2%(w/v) and 4%(w/v), preferably 3%(w/v), of Poloxamer 188 (Kolliphor P188);
c) between 20%(w/v) and 25%(w/v), preferably 23%(w/v), of Poloxamer 407 (Kolliphor P407);
d) water or phosphate buffer.

11. Brain-Derived Neurotrophic Factor (BDNF) for use as claimed in claim 1 to 10, wherein said hearing loss is measured by an audiometry test.

12. Brain-Derived Neurotrophic Factor (BDNF) for use as claimed in claim 11, wherein no hearing loss is present in subjects that in an audiometry test detect sounds below 25 dB at frequencies of 250-8,000 Hz.

13. Brain-Derived Neurotrophic Factor (BDNF) for use as claimed in claims 1 to 12, wherein said prevention of a hearing loss is determined by comparing the hearing function of the patient after administration of the platinum-based chemotherapy drug to the baseline hearing function of the subject prior to administration of the platinum-based chemotherapy drug.

14. Brain-Derived Neurotrophic Factor (BDNF) for use as claimed in claims 1 to 13, wherein said BDNF is human BDNF, more preferably it is recombinant human BDNF

15. Brain-Derived Neurotrophic Factor (BDNF) for use as claimed in claims 1 to 14, wherein said BDNF has a purity higher than 70%, more preferably higher than 80%, higher than 90%, higher than 95%, higher than 98% or higher than 99%.
